# EUROPÄISCHE PATENTANMELDUNG

(11) **EP 2 153 889 A2**
(43) Veröffentlichungstag der Anmeldung: **17.02.2010**
(21) Anmeldenummer: 09165855.9
(22) Anmeldetag: 20.07.2009
(51) Int. Cl.: B01F 17/42, A01N 25/04, A01N 43/653, A01N 53/00, B01F 17/54

(54) **Nanoemulsionen und Verfahren zu deren Herstellung, sowie deren Verwendung als Formulierungen von Pflanzenschutz- und/oder Schädlingsbekämpfungsmitteln und/oder kosmetischen Zubereitungen**

(30) Priorität: 15.08.2008 US 89079 P
(71) Anmelder: Evonik Goldschmidt GmbH, 45127 Essen (DE)
(72) Erfinder: Giessler-Blank, Sabine, Dr., 44227 Dortmund (DE); Scheuermann, Ralph, Dr., 45359 Essen (DE); Venzmer, Joachim, Dr., 45239 Essen (DE); Lindsay, David, Dr., Chester, VA 23836 (US)

(57) **Zusammenfassung**

Nanoemulsionen und Verfahren zu deren Herstellung, sowie deren Verwendung als Formulierungen von Pflanzenschutz- und/oder Schädlingsbekämpfungsmitteln und/oder kosmetischen Zubereitungen.

## Beschreibung

Ein kostengünstiges, speziell energieeffizientes Verfahren zur Herstellung feinteiliger Öl-in-Wasser Emulsionen, die eine hervorragende Stabilität in großen Temperatur- und Konzentrationsbereichen aufweisen, ist das sogenannte PSQ-(Phase shift by quenching)-Verfahren.
Das in EP-A2-1 882 516 beschriebene PSQ-Verfahren bietet sich für die Anwendung auf Pflanzenschutzmittelformulierungen an.
Bei diesem Verfahren wird in einer ersten Stufe die sogenannte Phaseninversionstemperatur (PIT) eines Mikroemulsionssystems durch Verwendung von kosmotropen Substanzen mindestens bis auf das Niveau der Raumtemperatur bzw. die Anwendungstemperatur gesenkt und in einer zweiten Stufe durch Zugabe von Verdünnungsmitteln wieder erhöht, vorzugsweise auf das ursprüngliche Niveau. Dabei bleibt die Emulsion erhalten, geht dabei von einer thermodynamisch stabilen Mikroemulsion in eine kinetisch stabile Nanoemulsion über.

Die Anwendung des PSQ-Verfahrens ist allerdings nicht in allen Fällen vorteilhaft, da oftmals pflanzenphysiologisch aktive Salze als kosmotrope Substanzen eingesetzt werden müssen.

Die kosmotropen Substanzen ersetzen im PSQ-Verfahren den Schritt der Temperaturerhöhung des PIT-Verfahrens (Phase Inversion temperature, K.Shinoda; H.Kunieda, Encyclopedia of Emulsion Technology; Vol 1 (1983), p. 337ff)). Bei dem PIT-Verfahren muss die Mischung der Komponenten über die Phaseninversionstemperatur aufgeheizt werden, um die bei Raumtemperatur vorliegende O/W-Emulsion in eine W/O-Emulsion umzukehren und durch anschließendes, rasches Abkühlen eine feinteilige O/W-Emulsion herzustellen. Der erforderliche Energieaufwand zur Aufheizung und effektiven Abkühlung ist der erhebliche Nachteil des PIT-Verfahrens und macht es daher weitgehend unökonomisch.

Durch Zugabe von ausreichenden Mengen an Verdünnungsmitteln, erfindungsgemäß vorzugsweise Wasser oder wässrige, gegebenenfalls alkoholische Lösungen, wird die für die Senkung der Phaseninversionstemperatur erforderliche Mindestkonzentration der kosmotropen Substanzen (KS) unterschritten, so dass das ursprüngliche Temperaturniveau wiederhergestellt wird. Die Verdünnung erfolgt dabei so schnell, dass - ähnlich wie bei der schnellen Temperatursenkung - keine Phasentrennung der resultierenden Nanoemulsion erfolgt.
Unter kosmotropen Substanzen versteht man solche Substanzen, die die Ausbildung von Wasserstoffbrückenbindungen in einer wässrigen Phase begünstigen bzw. verstärken. Durch Zusatz einer kosmotropen Substanz kann somit die Hydrophilie/Hydrophobie einer amphiphilen Substanz in Richtung stärkerer Hydrophobie verschoben werden.
Durch Verwendung von kosmotropen Substanzen wird die Temperatur einer der Mikroemulsionsphasengrenzen oder gar beider Temperaturen der Mikroemulsionsphasengrenzen auf ein tieferes Niveau gesenkt.
Beispiele kosmotroper Substanzen sind in der EP-A2-1 882 516 erwähnt, gemein ist diesen wasserlöslichen Substanzen entweder ein ionischer, salzartiger Aufbau oder aber die Zugehörigkeit zu der Gruppe der ebenfalls wasserlöslichen organischen Säuren oder Alkohole.

Eine Gemeinsamkeit des PSQ- und des PIT-Verfahrens besteht darin, dass die nach Verdünnung der Mikroemulsion erhaltene Nanoemulsion hinreichend "weit" von der Phasengrenze zum Mikroemulsionsgebiet entfernt sein sollte und damit "eingeforen" bestehen bleibt. Wird eine Temperatur nahe der Phasengrenze erreicht, so zerfällt die Nanoemulsion unter Bildung großer Tropfen und letztendlich tritt Phasenseparation auf. Im Vergleich zu Mikroemulsionen und Nanoemulsionen benötigen (Standard-)Emulsionen zu ihrer Herstellung vergleichsweise große Mengen an Energie, und während sie eine eingeschränkte kinetische Stabilität aufweisen besitzen sie keine thermodynamische Stabilität.

Weitere Verfahren zur Herstellung von Nanoemulsionen, beispielsweise Mikrofluidisierung und Ultraschallbehandlung sind ebenfalls generell unattraktiv, da diese Verfahren sehr kostenträchtig und /oder mit einem erheblichen Energieaufwand verbunden sind. Siehe dazu beispielsweise Lawrence et al., "Recent Advances in Microemulsions as Drug Delivery Vehicles", nanoparticulates as Drug Carriers, Herausgeber V.P. Torchilin, Imperial College Press 2006.
Es bestand daher weiterhin der Bedarf nach einem kostengünstigen Verfahren, feinteilige Emulsionen, die eine hervorragende Stabilität in einem großen Temperatur- und Konzentrationsbereich aufweisen, herzustellen, dabei jedoch die Nachteile des PIT- und des PSQ-Verfahrens weitestgehend zu überwinden.

Ziel der zugrunde liegenden Erfindung war es somit, bestehende, auf der Verdünnung bzw. dem Abschrecken von Mikroemulsionen basierende Verfahren zur Herstellung von Nanoemulsionen so zu modifizieren, dass sie auf die Anwendung von starken Temperaturänderungen oder die Verwendung von kosmotropen Verbindungen zumindest weitgehend verzichten können und in einer weiteren Ausgestaltung auf die Formulierung von physiologisch wirksamen Zusammensetzungen, wie beispielsweise Pflanzenschutz- und/oder Schädlingsbekämpfungsmitteln und/oder kosmetischen Zubereitungen und/oder fungiziden Zubereitungen, zu überführen.

Überraschenderweise wurde nun gefunden, dass sich Nanoemulsionen durch Verdünnen von Mikroemulsionen herstellen lassen, in dem hydrophobe, schlecht oder gar wasserunlösliche und damit per se nicht-kosmotrope Substanzen verwendet werden, die in der Lage sind, in Bezug auf die Temperatur eine (kosmotrop-)analoge Wirkung zu erzielen und die mindestens eine Temperatur der Mikroemulsionsphasengrenzen auf ein tieferes Niveau zu senken vermögen.

Unter dem Begriff "pseudo-kosmotrop" und seiner Verwendung im Rahmen dieser Anmeldung ist eine Substanz zu verstehen, die hydrophob ist, daher nicht in der Wasserphase vorliegt und damit nicht als Kosmotrop bezeichnet werden kann, aber dennoch die Phasengrenzen einer Mikroemulsion wie ein Kosmotrop verschiebt, also in Bezug auf die Temperatur wie ein Kosmotrop wirkt.

Die erfindungsgemäßen pseudo-kosmotropen, hydrophoben Substanzen sind nicht nur in der Lage, die Phasengrenze einer Mikroemulsion abzusenken, sondern zusätzlich führen sie überraschenderweise auch dazu, dass die nach Verdünnung der Mikroemulsion erhaltenen Nanoemulsionen stabil sind.

Somit ist ein Gegenstand der Erfindung ein Verfahren zur Herstellung einer Nanoemulsion, wobei mindestens eine hydrophobe pseudo-kosmotrope Substanz, mindestens eine hydrophile Substanz, mindestens ein Emulgator, gegebenenfalls ein oder die Mischung mehrerer hydrophober Öle, sowie gegebenenfalls weitere Adjuvantien oder Hilfsmittel zu einer Mikroemulsion miteinander vermischt und danach zur Ausbildung der Nanoemulsion verdünnt werden.

Es kann damit auf den Zusatz zusätzlicher Anionen/Kationen oder anderer organischer Verbindungen, die als Kosmotrop gelten, ganz verzichtet oder zumindest deren Konzentration reduziert werden.
Kosmotrope Substanzen können allerdings gegebenenfalls (weiterhin) mitverwendet werden, um beispielweise das Temperaturprofil in den Mikroemulsionen unter anwendungstechnischen Gesichtspunkten zu optimieren oder gegebenenfalls in ihrer Funktion als Mikronährstoffe die Emulsion zu bereichern.

Im Gegensatz zu kosmotropen Substanzen, die in der Wasserphase vorliegen, sind die erfindungsgemäßen pseudo-kosmotropen Substanzen nicht wasserlöslich und liegen in der Ölphase vor. Nicht oder schlecht wasserlöslich bedeutet im Rahmen dieser Erfindung eine Löslichkeit in Wasser von weniger als 1,0 g/l bei 25° C, vorzugsweise weniger als 0,5 g/l und ganz besonders bevorzugt von weniger als 0,1 g/l.

Interessanterweise fallen unter diese überraschend aufgefundenen pseudo-kosmotropen Substanzen auch beispielsweise viele Pflanzenschutz- und/oder Schädlingsbekämpfungswirkstoffe, speziell Fungizide und Insektizide.

Überraschenderweise wurde gefunden, dass beispielsweise Pestizide oder Pestizidgemische, die selbst schlecht oder gar nicht in Wasser löslich sind, eine Verschiebung der Phasengrenze der Mikroemulsion ebenso wie ein in der Wasserphase befindliches Kosmotrop bewirken und so die Anwendung eines Verfahrens analog des PSQ-Verfahrens ermöglichen, d.h. vorteilhaft zur Formulierung von Mikroemulsionen geeignet sind, aus denen dann nachfolgend durch Verdünnung Nanoemulsionen hergestellt werden können.

Besonders überraschend ist die Tatsache, dass zu den vielen hydrophoben Substanzen gerade auch die physiologisch wirksamen Substanzen oder Zusammensetzungen gehören, die es als pseudo-kosmotrope Substanzen vermögen, mindestens eine der Temperaturen der Mikroemulsionsphasengrenze auf ein tieferes Niveau zu senken.

**Zur Anwendung kommen Pestizid** wirkstoffe oder Wirkstoffgemische, zum Beispiel Herbizide, Fungizide, Wachstumsregulatoren, Molluskizide, Mikronährstoffe sowie Insektizide, welche schlecht bzw. nicht in Wasser löslich sind, bevorzugt mit einer Wasserlöslichkeit von weniger als 1,0 g/l, insbesondere kleiner als 0,5 g/l bei 25°C und besonders bevorzugt von weniger als 0,1 g/l.
Die c h e mischen Klassen und Bestandteile oder die Zusammensetzung von Wirkstoffen in Zusammenhang mit Ihrer Verwendung und Einsatzgebieten sind z. B. im 'The Pesticide Manual', 14th edition, 2006, The British Crop Protection Council, oder im 'The Manual of Biocontrol Agents', 2001, The British Crop Protection Council, aufgelistet."
Diese Stoffe oder Gemische können weitere Komponenten enthalten, die üblicherweise bei der Herstellung von Pestizidzusammensetzungen mitverwendet werden.
Nicht abschließend und nur beispielhaft genannt seien Pestizide der folgenden Stoffklassen: Pyrethroide, Sulfonylharnstoffe, Triazole, Morpholine, Phenylpyrazole, Neo-nicotinoide, Tetracycline, Cyclodiene, Organochlorine, Organophosphor basierte Pestizide, Carbamate und Dithiocarbamate, Phthalimide, Strobilurine, Benzimidazole, Aryloxyphenoxypropionate, und/oder Triazine.
Insbesondere erfasst von dem erfindungsgemäßen Verfahren sind Abamectin (Wasserlöslichkeit 7-10 µg/l), Tebuconazol (Wasserlöslichkeit 36 mg/l), Nicosulfuron (Wasserlöslichkeit 0,07 g/l) , Niclosamid (Wasserlöslichkeit 0,1 g/l), Tridemorph (Wasserlöslichkeit 1,1 mg/l), Epoxyconazol (Wasserlöslichkeit 6,63*10⁻⁴ g/100ml), Bifenthrin (Wasserlöslichkeit <1µg/l), Permethrin (Wasserlöslichkeit 6x10⁻³ mg/l), Fipronil (Wasserlöslichkeit 1,9 mg/l), Chlorpyrifos (Wasserlöslichkeit 1 mg/l), Endosulfan (Wasserlöslichkeit 0,32 mg/l), Mancozeb (Wasserlöslichkeit 6,2 ppm), Captan (Wasserlöslichkeit 3,3 mg/l), Azoxystrobin (Wasserlöslichkeit 6 mg/l), Carbendazim (Wasserlöslichkeit 29 mg/l), Clodinafop-propargyl (Wasserlöslichkeit 4 mg/l), Atrazin (Wasserlöslichkeit 33 mg/l).

Um für den Anwender bezüglich Kennzeichnung und Geruch unangenehme Lösemittel zu vermeiden, war es das Hauptanliegen, leicht herzustellende und mit Wasser beliebig verdünnbare Mikroemulsionen zur Darstellung der für Spritzbrühen verwendbaren Nanoemulsionen herzustellen.

Im Pflanzenschutz werden üblicherweise zur Verbesserung der Effizienz sogenannte Adjuvantien oder auch Hilfsmittel eingesetzt. Diese werden entweder der wässrigen Spritzbrühe kurz vor dem Aufsprühen zugesetzt (als Tankmischungsadditiv) oder direkt in Formulierungen eingebaut. Tankmischungsadditive setzen die Oberflächenspannung von Spritztropfen herab. Diese verringerte Oberflächenspannung gegenüber Wasser (72 mN/m) bewirkt ein besseres Eindringen der Formulierung durch den hydrophoben Blattkörper, d.h. Pestizide wirken besser und werden stärker, speziell von Pflanzen, aufgenommen. Trisiloxane und Polysiloxane sind dafür bekannt, dass sie die Oberflächenspannung auf <30 mN/m verringern können.
Um den zusätzlichen Arbeitsschritt des Tankmischungsadditives einzusparen, war ein weiterer Gegenstand der Erfindung, diese Adjuvantien - auch als Spreiter bekannt - in die Mikroemulsionen einzuarbeiten.

Für die in den Mikroemulsionen verwendeten Adjuvantien haben sich sogenannte Ölspreiter als vorteilhaft erwiesen. Diese Ölspreiter kommen aus der Klasse der organisch modifizierten Polysiloxane, der allgemeinen Formel (1): 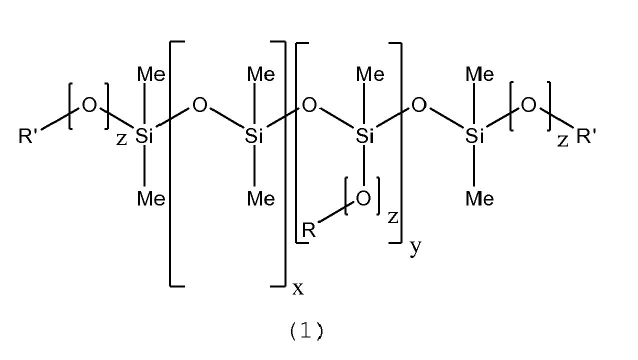 wobei
z = unabhängig voneinander 0 oder 1,
x = 0 bis 200, vorzugsweise 5 bis 150 und besonders bevorzugt 10 bis 100,
y = 0 bis 100, vorzugsweise 1 bis 35,
R' = R oder ein Alkylrest,
R = (CH₂)ₙ-(O)ₒ-(C₂H₄O)ₚ(C₃H₆O)_{q}(C₂H₃LO)_{r-K} und/oder CₘH₂ₘ₊₁
m = 1 bis 40, vorzugsweise 1 bis 30 und besonders bevorzugt 8 bis 24,
n = 0, 3, 4 oder 6, vorzugsweise 0 oder 3
o = 0 oder 1
p = 0 bis 50, vorzugsweise 0 bis 25,
q = 0 bis 50, vorzugsweise 0 bis 25,
r = 0 bis 50, vorzugsweise 0,
L = Ethyl- oder Phenyl,
K gleich H, ein Alkylrest mit 4 oder weniger Kohlenstoffatomen oder eine Acetylgruppe ist,
mit der proviso, dass das Molekül mindestens eine Gruppe R enthalten muss.

Als weitere Adjuvantien im Rahmen dieser erfindungsgemäßen Zusammensetzungen können auch kosmotrope Substanzen mitverwendet werden, die beispielsweise zur Optimierung des Temperaturprofils der Gemische unter anwendungstechnischen Gesichtspunkten beitragen.

Ein wesentlicher Unterschied zwischen Mikroemulsionen und Nanoemulsionen ist der Stabilitätsgrad und Typus. Während Mikroemulsionen thermodynamisch stabil sind, sind Nanoemulsionen (manchmal auch als Mini- oder submikron-Emulsionen benannt) kinetisch stabil.
Eine Mikroemulsion kann daher mit nahezu beliebigen Methoden hergestellt werden, während eine Nanoemulsionbesondere Methoden erfordert. Bei der Typbestimmung beider Emulsionen kann man sich das Verhalten bei Temperaturvariation zunutze machen. Werden beide Emulsionstypen auf hohe Temperaturen aufgeheizt und dann wieder abgekühlt, so wird sich die Nanoemulsion aufspalten während die Mikroemulsion als thermodynamisch stabile durch einfaches Schütteln wieder in die Ursprungsform überführen lässt.

Die Stabilität der Mikroemulsionen sollte in einem Temperaturbereich zwischen -10°C und +90°C, bevorzugt zwischen 0°C und 70°C und besonders bevorzugt zwischen +5°C und 60°C gegeben sein.
Stabilität bedeutet hier, dass keine Kristallisation und keine Phasentrennung auftritt, also immer ein 1-Phasensystem vorliegt.

Der Zeitraum über den Stabilität der Nanoemulsion gegeben sein muss, beträgt mindestens 24 Stunden, bevorzugt mindestens 48 Stunden unter Anwendungsbedingungen, jeweils bei einer Temperatur von zumindest 25°C.
Die Mikroemulsion soll mindestens 2 Jahre, bevorzugt mindestens 3 Jahre und ganz bevorzugt 5 Jahre bei 25 °C lagerstabil sein.

Der Temperaturbereich ist bezüglich Lagerung und Transport bevorzugt, jedoch auch durch die Prüfmethode CIPAC MT 46, die die Überprüfung der Stabilität von Pflanzenschutzmittelformulierungen beschreibt, zugrunde gelegt. Die durch Verdünnen mit Wasser herstellbaren Nanoemulsionen weisen Partikelgrößen zwischen 10 und 300, bevorzugt zwischen 20 und 250 nm und besonders bevorzugt zwischen 30-200 nm auf.

Ein weiterer Gegenstand der vorliegenden Erfindung ist ein Verfahren zur Herstellung einer Nanoemulsion durch Verdünnung einer Mikroemulsion, die mindestens eine hydrophobe, pseudo-kosmotrope Substanz im Sinne der Erfindung enthält, umfassend
A) Herstellen einer Mischung 2, die mindestens eine pseudo-kosmotrope Substanz, Wasser, gegebenenfalls ein organomodifiziertes Polysiloxan, gegebenenfalls eine hydrophobe Substanz und mindestens einen Emulgator aufweist,
   wobei mindestens eine der Phasengrenzen der Mikroemulsionsphase dieser Mischung bei einer tieferen Temperatur liegt als die entsprechende Phasengrenze der Mikroemulsionsphase einer Mischung 1, die keine pseudo-kosmotrope Substanz und ansonsten die gleiche Zusammensetzung wie Mischung 2 aufweist,
   und nachfolgend einen Schritt
B) Zugabe eines Verdünnungsmittels zur Mischung 2 zur Überführung dieser Mischung in eine Nanoemulsion 3, wobei die Menge des zugegebenen Verdünnungsmittels so gewählt ist, dass die erhaltene Nanoemulsion 3 bei einer vorgegebenen Temperatur nicht als Mikroemulsionsphase vorliegt.

Ein weiterer Gegenstand der vorliegenden Erfindung sind Nanoemulsionen, bei denen die zugrundeliegenden Mikroemulsionen die pseudo-kosmotrope Substanz in einer Menge von 0,5 bis 40 Gew.-%, bevorzugt mit 2 bis 30 Gew.-%, besonders bevorzugt mit 3 bis 25 Gew.-% enthalten.

Ein weiterer Gegenstand der vorliegenden Erfindung sind Nanoemulsionen, bei denen die zugrundeliegenden Mikroemulsionen neben der pseudo-kosmotropen Substanz als weitere hydrophobe Substanz ein Öl oder mehrere Öle enthalten, die mindestens einen Flammpunkt von 110°C haben und aus der Gruppe der Mineralöle, aromatischen Öle, pflanzlichen Öle, Fettsäurester, Paraffinöle oder Siliconöle stammen.
Bevorzugt werden dabei für das verwendete Öl hydrophobe Öle, Aliphaten aus der Gruppe der Mineralöle sowie Paraffinöle oder deren Mischungen verwendet.

Ein weiterer Gegenstand der vorliegenden Erfindung sind Nanoemulsionen, bei denen die zugrundeliegenden Mikroemulsionen ein Additiv auf Basis eines organo-modifizierten Polysiloxans der allgemeinen Formel (1) enthalten.

Ein weiterer Gegenstand der vorliegenden Erfindung sind Nanoemulsionen, bei denen die zugrundeliegenden Mikroemulsionen im Temperaturbereich -10°C und +90°C, speziell 0°C bis 70°C, ganz besonders +5°C und 60°C stabil sind.

Ein weiterer Gegenstand der vorliegenden Erfindung sind Nanoemulsionen, die beim Verdünnen einer Mikroemulsion mit Wasser gebildet werden, deren durchschnittliche Partikelgrössenverteilung im Bereich von 10-300nm, speziell 20-250nm, ganz besonders bevorzugt zwischen 30-200nm liegt.

Ein weiterer Gegenstand der vorliegenden Erfindung sind Nanoemulsionen, die durch beliebiges Verdünnen von Mikroemulsionen im Verhältnis von 1:3 bis 1:10000, bevorzugt 1:15 bis 1:1000, besonders bevorzugt 1:20 bis 1:500, bezogen auf Massenteile, mit Wasser herstellbar sind, die in der gebildeten Nanoemulsion Teilchengrössen aufweisen, die eine Transluzenz hervorrufen.

Ein weiterer Gegenstand der vorliegenden Erfindung ist die Verwendung der erfindungsgemäßen Nanoemulsionen enthaltend eine pseudo-kosmotrope Substanz, die zu den Pestiziden zählt und im crop- und non-crop-Bereich verwendet wird. Die Verwendung der englisch-sprachigen Begriffe erfolgt hier, da es sich um eingeführte, dem Fachmann wohlbekannte Begriffe handelt, die keine Entsprechung in der deutschen Sprache haben.
Unter crop wird die Behandlung von (Nutz-) Pflanzen im erweiterten Agrarumfeld verstanden, unter non-crop die Anwendung im Heim und Gartenbereich, sowie die Schädlingsbekämpfung beispielsweise an/in Holzgütern oder auch beispielsweise gegen Läusebefall.

Ein weiterer Gegenstand der vorliegenden Erfindung sind Nanoemulsionen, bei denen die zugrundeliegende Mikroemulsion beispielsweise Pestizide als pseudo-kosmotrope Substanzen aus der Gruppe der Pyrethroide (Konzentration in der Formulierung 0,5-40 Gew.-%), Sulfonylharnstoffe (Konzentrationgrenze in der Formulierung 0,5-25 Gew.-%), Triazole (Konzentration in der Formulierung 0,5-25 Gew.-%), Neo-nicotinoide (Konzentration in der Formulierung 0,5-20 Gew.-%) enthalten.

Ein weiterer Gegenstand der vorliegenden Erfindung sind Nanoemulsionen, bei denen das in der zugrundeliegenden Mikroemulsion enthaltende Pestizid oder Pestizidgemisch ein Insektizid ist.

Ein weiterer Gegenstand der vorliegenden Erfindung ist die Verwendung einer, ein oder mehrere Pestizide enthaltenden Nanoemulsion durch Verdünnen einer Mikroemulsion, hergestellt nach einem der vorbenannten erfinderischen Verfahren als Konzentrat zur Mitverwendung in veterinärischen und/oder pharmazeutischen und/oder kosmetischen Zubereitungen, beispielsweise zur Läusebekämpfung.

Die EP-A2-1 882 516 beschreibt unter dem Begriff einer Mikroemulsion eine thermodynamisch stabile Emulsion. Verdünnt man diese Mikroemulsion schnell mit Wasser, so ist die resultierende Lösung nicht mehr thermodynamisch, sondern nur noch kinetisch stabil. Der Impuls der kleinen in der Mikroemulsion schon vorhandenen Tröpfchen reicht beim Zusammenstoß nicht aus, eine Koaleszenz zu bewirken, so dass keine Phasentrennung auftritt, sondern eine Emulsion erhalten bleibt; wegen der kleinen Tröpfchen im Nanometerbereich spricht man von Nanoemulsionen.

Der **Vorteil in einer Pflanzenschutzmittelformulierung,** speziell einer Mikroemulsion und der daraus hergestellten Nanoemulsion, kein zusätzliches Kosmotrop einsetzen zu müssen, ist zum einen, dass die in EP-A2-1 882 516 genannten ionischen Verbindungen im Pflanzenschutz zum Teil als Mikronährstoffe verwendet werden. Speziell bei Applikationen in denen diese Kosmotrope nicht für die Pflanze benötigt werden, erzeugen sie zu hohe Werte, beispielsweise an Phosphat-Gehalt; dies kann zu Unverträglichkeit bei Pflanzen, gegebenenfalls sogar zu phytotoxischen Reaktionen führen.

Weiterhin kann auf einen Großteil der Emulgatormenge gegenüber bekannten Verfahren und Formulierungen verzichtet werden und da die Phaseninversionstemperatur abgesenkt ist, trotz des genannten Verzichts eine sehr schnelle Emulgierung erreicht werden. Die Emulgierung erfolgt bei den erfindungsgemäßen Systemen schnell und bereits unter Verzicht auf Mischanlagen, in denen unter der Wirkung hoher Scherkräfte die Emulgierung erfolgt.

Die Verwendung kosmotroper Substanzen ist in vielen Anwendungen nachteilig, da viele Pestizide ionisch wirken, und damit der Zusatz solcher Kosmotropen zu Inkompatibilitäten, z.B. Ausfällungen in der Formulierung führen kann.

Bei den erfindungsgemäßen Formulierungen besteht weiterhin der Vorteil, dass, wenn man keine zusätzlichen Kosmotrope benötigt, innerhalb der Formulierung mehr Raum für andere Komponenten ist und man recht konzentrierte Formulierungen herstellen kann. Auch kann der Wirkstoff, das sogenannte Active, in wesentlich höheren Konzentrationen in der Formulierung vorliegen. Dieses ist vorteilhaft hinsichtlich des Transportvolumens, als auch der Vermeidung des Ausbringens von unnötigen Chemikalien, was aus Umweltaspekten zu befürworten ist.

Mikroemulsionen, die für Agrochemikalien eingesetzt werden können, werden in PCT/US2004/007388 beschrieben. Die dort beschriebenen lipophilen Emulgatoren, die oft im Bereich Kosmetik verwendet werden, sind teuer und sollten daher in der hier zugrunde liegenden Mikroemulsion nicht verwendet werden. Es zeigte sich auch, dass die Verwendung von lipophilen Co-Emulgatoren bei dem in dieser hier vorliegenden Erfindung und dem beschriebenen Verfahren nicht notwendig ist.
Bei den dieser Erfindung zugrunde liegenden Emulgatoren werden vermehrt ethoxylierte Fettalkohole allein oder in Mischungen verwendet. Typische Emulgatoren sind TEGO Alkanol L 4 (Alkohol, C₁₂/C₁₄, ethoxyliert), Rewopal LA 6 (Laurylalkoholpolyethoxylat, n = 6), Rewopal LA 10 (Laurylalkoholpolyethoxylat, n = 10), Rewopal LA 12 - 80 (wässrige Lösung von Laurylalkoholpolyethoxylat, n = 12), TEGO Alkanol L 23 P (Laurylalkoholpolyethoxylat, n = 23). Diese weisen eine ausgeprägte - für die Anwendung des PSQ-Verfahrens erforderliche - Temperaturabhängigkeit auf, da die Hydrophilie/Hydrophobie des Emulgators vom Ausmaß der ausgebildeten Wasserstoffbrückenbindungen in der wässrigen Phase abhängt. Mit steigender Temperatur nimmt auch die Zahl der Wasserstoffbrückenbindungen ab, so dass der Emulgator weniger hydrophil wird und sich der Charakter der Emulsion von o/w zu w/o verschiebt.
Im Vergleich zu Alkylpolyglycosiden (APGs), wie sie in PCT/US2004/007388 verwendet werden, haben diese eine wesentlich geringere Schaumneigung, was bei der Anwendung der wässrigen Spritzbrühe wichtig ist. APGs hingegen werden meist beim Herstellen der Spritzbrühe zusätzlich mit Entschäumern kombiniert.

Bei Bedarf ist der Zusatz von Entschäumern zu der in dieser Erfindung beschriebenen Mikroemulsionsherstellung möglich.

Das Verfahren zur Herstellung einer erfindungsgemäßen Zusammensetzung erfolgt durch einfaches Rühren einer Mischung, die mindestens eine hydrophile Substanz, beispielsweise Wasser, mindestens eine hydrophobe, pseudo-kosmotrope Substanz, beispielsweise eine (pflanzen-, tier-, insekten- oder auch pilz-)physiologisch wirksame Substanz, gegebenenfalls ein oder mehrere hydrophobe Öle und/oder gegebenenfalls ein organomodifiziertes Polysiloxan sowie mindestens einen Emulgator aufweist.
Hierbei liegt mindestens eine Temperatur der Mikroemulsionsphasengrenzen dieser Mischung bei einer tieferen Temperatur als die entsprechende Phasengrenze der Mikroemulsionsphase einer Mischung, die keine physiologisch wirksame, pseudo-kosmotrope Substanz enthält. Bei der so erhaltenen erfindungsgemäßen Formulierung handelt es sich um eine Mikroemulsion. Durch Zugabe eines Verdünnungsmittels, beispielsweise Wasser, zu einer solchen Mikroemulsion wird diese in eine Nanoemulsion überführt.

Grundsätzlich sind als Emulgatoren alle Verbindungen, wie sie im Stand der Technik als Emulgatoren zur Herstellung von O/W- und W/O-Emulsionen verwendet werden, geeignet. Bevorzugt wird dabei mindestens ein Emulgator aus der Gruppe der ionischen und nichtionischen Emulgatoren eingesetzt.

Ohne Anspruch auf Vollständigkeit seien aus den bekannten Stoffklassen geeigneter Emulgatorkomponenten zusätzlich die folgenden Vertreter benannt:

Als nichtionische Emulgatoren kommen dabei besonders Oligo-Alkoxylate mit lipophile Reste enthaltenden Grundmolekülen in Frage. Diese können sich insbesondere von ausgewählten Vertretern aus den nachfolgenden Klassen der lipophile Reste enthaltenden Grundmoleküle ableiten: Fettalkohole, Fettsäuren, Fettamine, Fettamide, Fettsäure- und/oder Fettalkoholester und/oder -ether, Alkanolamide, Alkylphenole und/oder deren Umsetzungsprodukte mit Formaldehyd sowie weitere Umsetzungsprodukte von lipophile Reste enthaltenden Trägermolekülen mit niederen Alkoxiden. Wie angegeben, können die jeweiligen Umsetzungsprodukte auch wenigstens anteilsweise endgruppenverschlossen sein. Beispiele für Partialester und/oder Partialether mehrfunktioneller Alkohole sind insbesondere die entsprechenden Partialester mit Fettsäuren, beispielsweise von der Art der Glycerinmono- und/oder -diester, beispielsweise Rizinusöl-Oleat, Glykolmonoester, entsprechende Partialester oligomerisierter mehrfunktioneller Alkohole, Sorbitanpartialester und dergleichen sowie entsprechende Verbindungen mit Ethergruppierungen. Solche Partialester und/oder -ether können insbesondere auch Grundmoleküle für eine (Oligo)-Alkoxylierung sein.

Bei der Alkoxylierung finden vorzugsweise Ethylenoxid, Propylenoxid, Butylenoxid oder Styroloxid Anwendung.

Besonders bevorzugte nichtionische alkoxylierte Emulgatoren sind:
Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe;
Glycerinmono- und -diester und Sorbitanmono- und -diester von gesättigten und ungesättigten Fettsäuren mit 6 bis 22 Kohlenstoffatomen und deren Ethylenoxidanlagerungsprodukte;
Alkylmono- und -oligoglycoside mit 8 bis 22 Kohlenstoffatomen im Alkylrest und deren ethoxylierte Analoga.
Die Anlagerungsprodukte von Ethylenoxid und/oder von Propylenoxid an Fettalkohole, Fettsäuren, Alkylphenole, Glycerinmono- und -diester sowie Sorbitanmono- und -diester von Fettsäuren oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/oder Propylenoxid und Substrat, mit denen die Anlagerungsreaktion durchgeführt wird, entspricht; kammartig oder endständig modifizierte Siliconpolyether, wie sie etwa durch Hydrosilylierungsreaktionen unter bekannten Bedingungen durch Anlagerung alkenfunktionalisierter Polyether mit bevorzugt 2 bis 100 Mol Ethylenoxid und/oder Propylenoxid erhältlich sind. Die endständigen Hydroxylgruppen solcher Polyether können dabei gegebenenfalls auch alkylterminiert sein (insbesondere methylterminiert).

Weiterhin können als nichtionische Emulgatoren auch Verwendung finden:
Polyol- und insbesondere Polyglycerinester wie z.B. Polyglycerinpolyricinoleat oder Polyglycerinpoly-12-hydroxystearat. Ebenfalls geeignet sind Gemische von Verbindungen aus mehreren dieser Substanzklassen;
Partialester auf Basis linearer, verzweigter, ungesättigter bzw. gesättigter C₆/C₂₂-Fettsäuren, Ricinolsäure sowie 12-Hydroxystearinsäure und Glycerin, beispielsweise Glycerinmonodioleat, Polyglycerin, Pentaerythrit, Dipentaerythrit, Zuckeralkohole (z.B. Sorbit), Alkylglucoside (z.B. Methylglucosid, Butylglucosid, Laurylglucosid) sowie Polyglucoside (z.B. Cellulose);
Polysiloxan-Polyalkyl-Polyether-Copolymere bzw. entsprechende Derivate;
C₈/C₁₈-Alkylmono- und -oligoglycoside, ihre Herstellung und ihre Verwendung als oberflächenaktive Stoffe sind beispielsweise aus US 3,839,318, US 3,707,535, US 3,547,828, DE-OS 19 43 689, DE-OS 20 36 472 und DE-Al 30 01 064 sowie EP-A 0 077 167 bekannt. Ihre Herstellung erfolgt insbesondere durch Umsetzung von Glucose oder Oligosacchariden mit primären Alkoholen mit 8 bis 18 C-Atomen.

Als Emulgatoren mit ionischem Charakter kommen anionische, kationische und zwitterionische Emulgatoren in Frage. Anionische Emulgatoren enthalten wasserlöslich machende anionische Gruppen wie z.B. eine Carboxylat-, Sulfat-, Sulfonat- oder Phosphat-Gruppe und einen lipophilen Rest. Anionische Tenside sind dem Fachmann in großer Zahl bekannt und im Handel erhältlich. Dabei handelt es sich insbesondere um Alkylsulfate oder Alkylphosphate in Form ihrer Alkali-, Ammonium- oder Alkanolammoniumsalze, Alkylethersulfate, Alkylethercarboxylate, Acylsarkosinate sowie Sulfosuccinate und Acylglutamate in Form ihrer Alkali- oder Ammoniumsalze. Auch können Di- und Trialkylphosphate sowie Mono-, Di- und/oder Tri-PEG-alkylphosphate und deren Salze eingesetzt werden.

Auch kationische Emulgatoren können eingesetzt werden. Als solche sind insbesondere quaternäre Ammoniumverbindungen verwendbar, etwa Alkltrimethylammoniumhalogenide wie z.B. Cetyltrimethylammoniumchlorid oder -bromid oder Behenyltrimethylammoniumchlorid, aber auch Dialkyldimethylammoniumhalogenide wie z.B. Distearyldimethylammoniumchlorid. Weiterhin können Monoalklyamidoquats wie z.B. Palmitamidopropyltrimethylammoniumchlorid oder entsprechende Dialkylamidoquats eingesetzt werden. Weiterhin können biologisch gut abbaubare quaternäre Esterverbindungen eingesetzt werden, bei denen es sich meist um quaternierte Fettsäureester auf Basis von Mono-, Di- oder Triethanolamin handelt. Weiterhin können Alkylguanidiniumsalze als kationische Emulgatoren eingesetzt sein.

Weiterhin können als Emulgatoren zwitterionische Tenside verwendet werden. Als zwitterionische Tenside werden solche oberflächenaktiven Verbindungen bezeichnet, die im Molekül mindestens eine quaternäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammoniumglycinate, beispielsweise das Kokosacylaminopropyldimethylammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat.
Besonders bevorzugt ist das unter der CTFA-Bezeichnung Cocamidopropylbetain bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C₈/C₁₈-Alkyl- oder C_{8/}C₁₈-Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂-C₁₈-Acylsarcosin. Neben den ampholytischen kommen auch quaternäre Emulgatoren in Betracht, wobei solche vom Typ der Esterquats, vorzugsweise methylquaternierte Difettsäuretriethanolaminester-Salze, besonders bevorzugt sind.

Es sei bei den ionischen Emulgatoren deutlich gemacht, dass ein Emulgator per definitionem an der Grenzfläche zwischen der hydrophilen und der hydrophoben Phase sitzt und somit keine kosmotrope Substanz sein kann. Kosmotrope Substanzen sind solche, die in der Wasserphase vorliegen und einen Einfluss auf die Ausbildung der Wasserstoffbrückenbindungen haben.

Besonders bevorzugt ist der Einsatz mindestens eines alkoxylierten nichtionischen Emulgators. Dieser nichtionische Basisemulgator oder die Kombination mehrer nichtionischer Emulgatoren kann in einer besonders bevorzugten Ausführung der Erfindung mit ionischen Emulgatorkomponenten kombiniert werden.

Allen Emulgatoren ist gemein, dass sie die Grenzfläche zwischen der hydrophilen und der hydrophoben Substanz, die zu emulgieren sind, bilden. Somit verhalten sie sich nicht wie typische kosmotrope Substanzen, die sich bevorzugt in der wässrigen Phase und nicht in der Grenzfläche aufhalten.

Bevorzugt verwendete hydrophobe Öle bzw. Lösemittel sind pflanzliche Öle, wie Rapsöl, Sonnenblumenöl, Rüböl, Sojaöl, Fettsäureester wie Rapssäuremethylester der Agnique^{®} Reihe sowie auch Methyloleat oder Methyllaurat oder auch Rohrzuckerester, aromatische Kohlenwasserstoffe wie C₁-C₆ Alkylbenzole (Toluol, Xylol), Testbenzin, C₉-C₁₂ Aromaten wie die Aromatic^{®} Reihe (Exxon), Isopar L und M, C₁-C₆ Alkylnaphthaline und Aromatengemische wie die Solvesso^{®} Reihe von Exxon, Mineralöle der Shellsol^{®} Reihe, Ketone wie Acetophenon, Isophoron, Cyclohexanon und Methylethylketon, aromatische, cycloaliphatische oder aliphatische Ether wie Tetrahydrofuron oder Methyl-tert butylether, N-substituierte C₁-C₁₂ Alkylpyrrolidone wie N-methylpyrrolidon, cyclische aliphatische Kohlenwasserstoffe sowie aliphatische Paraffinöle wie Kerosin oder auch OLEO FC^{®} · Zudem bieten sich Siliconöle an.
Es bieten sich jedoch auch Mischungen von Ölen/Lösemitteln an.

Werden die Nanoemulsionen gemäß der vorliegenden Erfindung in tierärztlichen, pharmazeutischen und/oder kosmetischen Zubereitungen eingesetzt, so können weitere Aktivwirkstoff/e nach dem Stand der Technik enthalten sein. Beispiele für solche Stoffe sind beschrieben in Plumb's Veterinary Drug Handbook, 5. Auflage 2005, Merck Index, 14. Auflage 2006 und dem International Cosmetic Ingredient Dictionay and Handbook, 12. Auflage 2008.

Die erfindungsgemäßen Formulierungen und das Verfahren zu deren Herstellung werden nachfolgend beispielhaft beschrieben, ohne dass die Erfindung als auf diese beispielhaften Ausführungsformen beschränkt angesehen werden kann. Sind nachfolgend Bereiche, allgemeine Formeln oder Verbindungsklassen angegeben, so sollen diese nicht nur die entsprechenden Bereiche oder Gruppen von Verbindungen umfassen, die explizit erwähnt sind, sondern auch alle Teilbereiche und Teilgruppen von Verbindungen, die durch Herausnahme von einzelnen Werten (Bereichen) oder Verbindungen erhalten werden können.

Weitere Ausgestaltungen des erfindungsgemäßen Verfahrens ergeben sich aus den Ansprüchen.

### Experimenteller Teil:

In den nachfolgend aufgeführten Beispielen wird die vorliegende Erfindung beispielhaft beschrieben, ohne dass die Erfindung, deren Anwendungsbreite sich aus der gesamten Beschreibung und den Ansprüchen ergibt, als auf die in den Beispielen genannten Ausführungsformen beschränkt gelesen werden kann. Alle Prozentangaben beziehen sich, wenn es nicht explizit anders angegeben ist, auf das Gewicht.
Verfahren zur Herstellung der Nano- und Mikroemulsion

### Beispiel 1a - Permethrin als pseudo-kosmotroper hydrophober Wirkstoff:

Herstellung einer Nanoemulsion durch Verdünnen einer Mikroemulsion, hergestellt durch einfaches Zusammenrühren der Komponenten in der Zusammensetzung wie sie in folgender Tabelle 1 angegeben ist:

| | Mikroemulsion [Gew.-%] | Nanoemulsion nach Verdünnung der Mikroemulsion zu 1:20 [Gew.-%] |
|---|---|---|
| Paraffinöl (dünnflüssiges Paraffinöl mit einem Siedepunkt von 260°C) | 36,97 | 1,85 |
| Wasser | 9,25 | 95,46 |
| Laurylalkoholpolyethoxylat (n = 6) | 29,57 | 1,47 |
| Laurylalkoholpolyethoxylat (n = 23) | 3,70 | 0,19 |
| Organomodifiziertes Polysiloxan (=Cetyl Dimethicon) | 0,18 | 0,01 |
| Permethrin | 20,33 | 1,02 |
| Gesamt | 100 | 100 |

### Charakterisierung:

Die am häufigsten auftretende Partikel- bzw. Größenverteilung der Tröpfchen (und alle im Weiteren diskutierten Größenverteilungen) wurden mit Hilfe von dynamischen Lichtstreumessungen (DLS) bestimmt. Alle Lichtstreumessungen wurden an verdünnten Proben mit ca. 0,5 Gew.-% Ölphasengehalt (wobei als Ölphase alle Komponenten, die nicht Wasser sind, bezeichnet werden) bei 25°C mit einem Malvern HPPS 3.1, Malvern Instruments Ltd, durchgeführt. Zur Beschreibung der Teilchengröße wird der hydrodynamische Radius rₕ verwendet.
Die am häufigsten auftretende Partikelgröße (Radius) der Nanoemulsion des zugrunde liegenden Beispiels liegt bei ungefähr 82 nm. Die Verdünnung der Mikroemulsion mit Wasser von 1:20 führt zu einer Nanoemulsion mit einem Pflanzenschutzmittelgehalt von ca. 1 Gew.-%, wie sie in Spritzbrühen verwendet wird. Eine weitere Verdünnung von 1:10 wird durchgeführt, um die Konzentration von allen nichtwässrigen Komponenten auf eine für die Lichtstreuung geeignete Konzentration von ca. 0,4 Gew.-% (allgemein ca. 0,1 - 1,0 Gew.-%) zu bringen.

Zur weiteren Charakterisierung der Mikroemulsion wurde die Oberflächenspannung in Substanz gemessen, also ohne Verdünnung vermessen. Die Oberflächenspannung wurde mittels der Methode des hängenden Tropfens (pendent drop) bestimmt (Messgerät: OCA 35 von Data Physics).
Die Formulierung nach Beispiel 1 zeigt hier eine Oberflächenspannung von 23,5 mN/m. Die gleiche Formulierung, jedoch ohne Polysiloxan als Adjuvant, zeigt einen Wert von 30,0 mN/m.

Die Bestimmung des Temperaturbereiches, in dem eine homogene, einphasige Mikroemulsionsphase vorliegt, erfolgt nach folgendem Verfahren: Beginnend bei Raumtemperatur (ca. 23 °C) wird das Mikroemulsionsgemisch mit einer Heizrate von ca. 3 °C/min erwärmt und mit Hilfe eines Magnetrührers bei moderaten Rührgeschwindigkeiten gerührt. Eine eventuell auftretende Phasentrennung äußert sich zunächst durch Ausbildung mikroskopisch feiner Tröpfchen, die zu einer Brechung des sichtbaren Lichts und somit zu einer makroskopisch sichtbaren Eintrübung des Systems führen. Beim anschließenden Abkühlen ändert sich das Erscheinungsbild des Gemisches. Bei Erreichen bzw. Unterschreiten der Temperatur, unterhalb derer das Mikroemulsionsgemisch in einer homogenen, einphasigen Mikroemulsionsphase vorliegt, wechselt es von trüb zu transparent.
Die Temperatur der unteren Phasengrenze des einphasigen Mikroemulsionsgebietes wurde durch Lagern für eine Stunde bei verschiedenen Temperaturen zwischen -5 °C und Raumtemperatur bestimmt.

Der Temperaturbereich der in Beispiel 1 beschriebenen Mikroemulsion liegt bei 5-80°C (Einphasengebiet). Wird die Mikroemulsion bei -5°C gelagert, so wird sie zwar fest, erlangt aber nach Erwärmen und Lagerung bei Raumtemperatur wieder ihre vorherige dünnflüssige, transparente Form.

Die Lagerstabilität der Nanoemulsion wurde durch Lichtstreuung nach Lagerung für drei Tage bei verschiedenen Temperaturen bestimmt. Bei Temperaturen bis 40°C trat eine leichte Zunahme der Tröpfchengröße auf ca. 100 nm auf. Bei 45°C erfolgte eine Zunahme auf ca. 200 nm, was als Zeichen einer beginnenden Koaleszenz der Tröpfchen interpretiert werden kann.

### Erfindungsgemäßes Beispiel 1b:

Verwendet man in der in Beispiel 1 beschriebenen Formulierung kein Laurylalkoholpolyethoxylat (n = 23) und läßt das Verhältnis der Konzentrationen der übrigen Komponenten konstant, so ist die Mikroemulsion von unter 5 °C bis 69 °C einphasig.

### Nicht erfindungsgemäßes Vergleichsbeispiel 1:

Verwendet man in der in Beispiel 1a beschriebenen Formulierung kein Permethrin, so ist die Mikroemulsion erst ab 69 °C (bis größer 80 °C) einphasig.

Das Permethrin in Beispiel 1a wirkt daher auf die Temperatur ähnlich wie ein Kosmotrop und senkt deutlich den Temperaturbereich der einphasigen Mikroemulsion ab.

### Beispiel 2 - Permethrin als pseudo-kosmotroper hydrophober Wirkstoff):

Herstellung einer Nanoemulsion durch Verdünnen einer Mikroemulsion, hergestellt durch einfaches Zusammenrühren der Komponenten in der Zusammensetzung wie sie in folgender Tabelle 2 angegeben ist:

| | Mikroemulsion [Gew.-%] | Nanoemulsion nach Verdünnung der Mikroemulsion zu 1:100 [Gew.-%] |
|---|---|---|
| Paraffinöl (dünnflüssiges Paraffinöl mit einem Siedepunkt von 260°C) | 44,45 | 0,44 |
| Wasser | 11,11 | 99,12 |
| Laurylalkoholpolyethoxylat (n = 6) | 31,11 | 0,31 |
| Permethrin | 13,33 | 0,13 |
| Gesamt | 100 | 100 |

### Charakterisierung:

Die Charakterisierung bezüglich der Emulsionstropfengröße erfolgte wie für Beispiel 1 beschrieben. Die am häufigsten auftretende Partikelgröße (Radius) der Nanoemulsion des zugrunde liegenden Beispiels liegt bei ungefähr 42 nm. Die Verdünnung der Mikroemulsion mit Wasser von 1:100 führt zu einer Nanoemulsion mit einem Wirkstoffgehalt von ca. 0,13 Gew.-%. Die Konzentration aller für die Lichtstreuung relevanten Komponenten lag bei ca. 0,9 Gew.-% (allgemein üblich ca. 0,1 - 1,0 Gew.-%).
Die Bestimmung des Temperaturbereiches, in dem eine homogene, einphasige Mikroemulsionsphase vorliegt, erfolgt nach dem für Beispiel 1 beschriebenen Verfahren. Der Temperaturbereich der in Beispiel 2 beschriebenen Mikroemulsion liegt bei 20-70°C (Einphasengebiet).

Verwendet man in der in Beispiel 2 beschriebenen Formulierung zusätzlich zu Laurylalkoholpolyethoxylat (n = 6) noch Laurylalkoholpolyethoxylat (n = 23) im Verhältnis 1**:**8 (n = 23 zu n = 6) und lässt das Verhältnis der Konzentrationen der übrigen Komponenten konstant, so ist die Mikroemulsion von 15 °C bis oberhalb 80 °C einphasig.

Vergleicht man die Beispiele 1 und 2, deren markantester Unterschied in der Permethrinkonzentration besteht (fast doppelte Konzentration in Beispiel 1), während die Gesamtemulgatorkonzentration konstant ist, so zeigt sich, dass Permethrin die Temperatur der oberen Phasengrenze des Einphasengebietes deutlich nach unten senkt und somit in Bezug auf die Temperatur einen einem Kosmotrop analogen Effekt bewirkt.

### Beispiel 3 - Tebuconazol als pseudo-kosmotroper hydrophober Wirkstoff:

Herstellung einer Nanoemulsion, erhalten durch Verdünnen einer Mikroemulsion, die durch einfaches Zusammenrühren der Komponenten in der Zusammensetzung wie sie in folgender Tabelle 3 angegeben ist hergestellt wurde:

| | Mikroemulsion [Gew.-%] | Nanoemulsion nach Verdünnung der Mikroemulsion zu 1:30 [Gew.-%] |
|---|---|---|
| Fettsäuretetraethylenpentamin | 37,74 | 1,26 |
| Wasser | 9,43 | 96,98 |
| Laurylalkoholpolyethoxylat (n = 6) | 37,74 | 1,26 |
| Tebuconazol | 15,09 | 0,50 |
| Gesamt | 100 | 100 |

### Charakterisierung:

Die Charakterisierung bezüglich der Emulsionstropfengröße erfolgt wie für Beispiel 1 beschrieben. Die am häufigsten auftretende Partikelgröße (Radius) der Nanoemulsion des zugrunde liegenden Beispiels liegt bei ungefähr 170 nm. Die Verdünnung der Mikroemulsion mit Wasser von 1:30 führt zu einer Nanoemulsion mit einem Wirkstoffgehalt von ca. 0,5 Gew.-%, entsprechend der Anwendungskonzentration für Spritzbrühen. Eine weitere Verdünnung von 1:6 wird durchgeführt, um die Konzentration von allen nichtwässrigen Komponenten auf eine für die Lichtstreuung geeignete Konzentration von ca. 0,5 Gew.-% (allgemein ca. 0,1 - 1,0 Gew.-%) zu bringen.
Die Bestimmung des Temperaturbereiches, in dem eine homogene, einphasige Mikroemulsionsphase vorliegt, erfolgt nach dem für Beispiel 1 beschriebenen Verfahren. Der Temperaturbereich der in Beispiel 3 beschriebenen Mikroemulsion liegt bei 5-80°C (Einphasengebiet).

### Beispiel 4: Vergleich der Nanoemulsionen erhalten durch das erfindungsgemäße Verfahren und erhalten durch das PIT-Verfahren

### 4a) erfindungsgemäßes Verfahren:

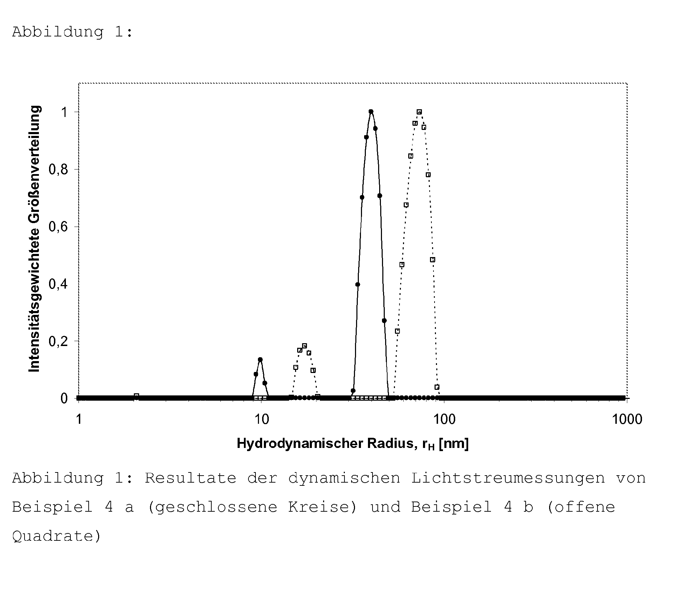

Die Charakterisierung bezüglich der Emulsionstropfengröße erfolgt wie für Beispiel 1 beschrieben. Die am häufigsten auftretende Partikelgröße (Radius) der Nanoemulsion des zugrunde liegenden Beispiels liegt bei 40 nm (siehe Abbildung 1, geschlossene Kreise). Die Verdünnung der Mikroemulsion mit Wasser von 1:100 führt zu einer Nanoemulsion mit einem Wirkstoffgehalt von 0,05 Gew.-%. Die Konzentration von allen nichtwässrigen Komponenten beträgt ca. 0,8 Gew.-% und ist somit eine für die Lichtstreuung übliche Konzentration (allgemein ca. 0,1 - 1,0 Gew.-%).
Die Bestimmung des Temperaturbereiches, in dem eine homogene, einphasige Mikroemulsionsphase vorliegt, erfolgt nach dem für Beispiel 1 beschriebenen Verfahren. Der Temperaturbereich der in Beispiel 4 a beschriebenen Mikroemulsion liegt bei 7-62 °C (Einphasengebiet).

### 4b) PIT-Verfahren:

Herstellung einer Nanoemulsion gemäß dem PIT-Verfahren durch einfaches Zusammenrühren der Komponenten und Erwärmen auf 80 °C bis in den einphasigen Mikroemulsionsbereich sowie nachfolgendes Abschrecken auf Raumtemperatur (ca. 23 °C).

Die Charakterisierung bezüglich der Emulsionstropfengröße erfolgt wie für Beispiel 1 beschrieben. Die am häufigsten auftretende Partikelgröße (Radius) der Nanoemulsion des zugrunde liegenden Beispiels liegt bei 74 nm (siehe Abbildung 1, offene Quadrate). Die weitere Verdünnung der Mikroemulsion durch Abschrecken mit Wasser im Massenverhältnis von 1:100 führt zu einer Nanoemulsion mit einem Wirkstoffgehalt von 0,05 Gew.-%. Die Konzentration von allen nichtwässrigen Komponenten beträgt ca. 0,8 Gew.-% und ist somit eine für die Lichtstreuung übliche Konzentration (allgemein ca. 0,1 - 1,0 Gew.-%).

### Fazit aus dem Vergleich von Beispiel 4a und 4b:

Mit dem erfindungsgemäßen Verfahren gelingt es energieeffizient eine lagerstabile Mikroemulsion herzustellen, deren Partikelgrößenverteilung kleiner ist als die mit der PIT Methode hergestellten Mikroemulsionen. Die daraus durch Verdünnung erhaltenen Nanoemulsionen sind geeignet als Formulierungen von Pflanzenschutz- und/oder Schädlingsbekämpfungsmi**tteln und /oder kosmetischen** Zubereitungen.
Abbildung 1 zeigt die Resultate der dynamischen Lichtstreumessungen.

## Patentansprüche

1. Verfahren zur Herstellung einer Nanoemulsion durch Verdünnen einer Mikroemulsion, **dadurch gekennzeichnet, dass** pseudo-kosmotrope, schlecht oder wasserunlösliche Substanzen mitverwendet werden, die mindestens eine Temperatur der Mikroemulsionsphasengrenzen auf ein tieferes Niveau senken.

2. Verfahren nach Anspruch 1, **dadurch gekennzeichnet, dass** mindestens eine hydrophobe pseudo-kosmotrope Substanz, mindestens eine hydrophile Substanz, mindestens ein Emulgator, gegebenenfalls ein oder die Mischung mehrerer hydrophober Öle und gegebenenfalls weitere Adjuvantien oder Hilfsmittel miteinander vermischt werden.

3. Verfahren nach zumindest einem der Ansprüche 1 oder 2, **dadurch gekennzeichnet, dass** als pseudo-kosmotrope, schlecht oder wasserunlösliche Substanz eine physiologisch wirksame Substanz oder Zusammensetzung verwendet wird.

4. Verfahren nach zumindest einem der Ansprüche 1 bis 3, **dadurch gekennzeichnet, dass** als Adjuvant ein oder mehrere Ölspreiter mitverwendet werden.

5. Verfahren nach Anspruch 4, **dadurch gekennzeichnet, dass** der Ölspreiter ein organisch modifiziertes Polysiloxan der allgemeinen Formel (1) ist. 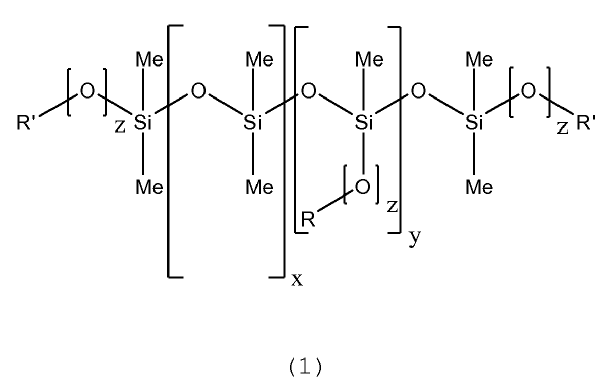 wobei
z = unabhängig voneinander 0 oder 1,
x = 0 bis 200,
y = 0 bis 100,
R' = R oder ein Alklyrest,
R = (CH₂)ₙ-(O)ₒ-(C₂H₄O)ₚ(C₃H₆O)_{q}(C₂H₃LO)_{r-K} und/oder CₘH₂ₘ₊₁
n = 0, 3, 4 oder 6,
m = 1 bis 40,
o = 0 oder 1
p = 0 bis 50,
q = 0 bis 50,
r = 0 bis 50,
L = Ethyl- oder Phenyl,
K gleich H, ein Alkylrest mit 4 oder weniger Kohlenstoffatomen oder eine Acetylgruppe ist,
mit der proviso, dass das Molekül mindestens eine Gruppe R enthalten muss.

6. Verfahren zur Herstellung einer Nanoemulsion nach zumindest einem der Ansprüche 1 bis 5 durch Verdünnen einer Mikroemulsion, die mindestens eine pseudo-kosmotrope Substanz enthält, umfassend
A) Herstellen einer Mischung 2, die mindestens eine pseudo-kosmotrope Substanz, Wasser, gegebenenfalls ein organomodifiziertes Polysiloxan, gegebenenfalls ein hydrophobe Substanz und mindestens einen Emulgator aufweist,
wobei mindestens eine der Phasengrenzen der Mikroemulsionsphase dieser Mischung bei einer tieferen Temperatur liegt als die entsprechende Phasengrenze der Mikroemulsionsphase einer Mischung 1, die keine pseudo-kosmotrope Substanz und ansonsten die gleiche Zusammensetzung wie Mischung 2 aufweist,
und nachfolgend einen Schritt
B) Zugabe eines Verdünnungsmittels zur Mischung 2 zur Überführung dieser Mischung in eine Nanoemulsion 3, wobei die Menge des zugegebenen Verdünnungsmittels so gewählt ist, dass die erhaltene Nanoemulsion 3 bei einer vorgegebenen Temperatur nicht als Mikroemulsionsphase vorliegt.

7. Nanoemulsion gemäß zumindest einem der Ansprüche 1 bis 6, **dadurch gekennzeichnet, dass** die pseudo-kosmotrope Substanz in einer Menge von 0,5 bis 40 Gew.-% der Gesamtformulierung der Mikroemulsion eingesetzt wird.

8. Nanoemulsion nach zumindest einem der Ansprüche 6 bis 7, wobei als hydrophobe Substanz ein Öl verwendet wird, das einen Flammpunkt von mindestens 110°C hat und zumindest ein Mineralöl, aromatishes Öl pflanzliches Öl, Fettsäureester, Paraffinöl oder Siliconöl, oder deren Mischungen enthält.

9. Nanoemulsion hergestellt nach einem Verfahren gemäß zumindest einem der Ansprüche 1 bis 6, **dadurch gekennzeichnet, dass** ein Adjuvant auf Basis eines organo-modifizierten Polysiloxans der allgemeinen Formel (1) 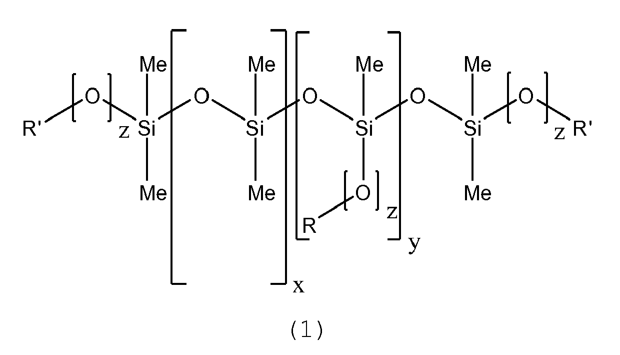 enthalten ist.

10. Nanoemulsion nach zumindest einem der Ansprüche 6 bis 9, bei der die zugrundeliegende Mikroemulsion in einem Temperaturbereich zwischen -10°C und +90°C stabil ist.

11. Nanoemulsion nach zumindest einem der Ansprüche 6 bis 10, **dadurch gekennzeichnet, dass** deren durchschnittliche Partikelgrössenverteilung im Bereich von 10-300nm liegt.

12. Nanoemulsion nach zumindest einem der Ansprüche 6 bis 11, **dadurch gekennzeichnet, dass** sie Teilchengrössen aufweisen, die eine Transluzenz hervorrufen.

13. Nanoemulsion hergestellt nach einem Verfahren gemäß zumindest einem der Ansprüche 1 bis 4 oder 6, **dadurch gekennzeichnet, dass** die pseudo-kosmotrope Substanz ein Pestizid oder Pestizidgemisch ist.

14. Nanoemulsion nach Anspruch 13, **dadurch gekennzeichnet, dass** die pseudo-kosmotrope Substanz ein Insektizid oder Insektizidgemisch ist.

15. Nanoemulsion nach zumindest einem der Ansprüche 7 bis 14, **dadurch gekennzeichnet, dass** als pseudo-kosmotrope Substanz zumindest ein Pestizid oder Insektizid oder deren Gemische aus den Stoffklassen der Pyrethroide, **Sulfonylharnstoffe, Triazole, Morpholine,** Phenylpyrazole, Neo-nicotinoide, Tetracycline, Cyclodiene, Organochlorine, Organophosphor basierte Pestizide, Carbamate und Dithiocarbamate, Phthalimide, Strobilurine, Benzimidazole, Aryloxyphenoxypropionate, und/oder Triazine verwendet werden.

16. Nanoemulsion nach zumindest einem der Ansprüche 7 bis 15, **dadurch gekennzeichnet, dass** eine kosmotrope Substanz mitverwendet wird.

17. Mikroemulsion nach einem Verfahren gemäß Anspruch 6, Verfahrensschritt A, **dadurch gekennzeichnet, dass** sie eine Lagerstabilität von mindestens 5 Jahren bei 25°C aufweist.

18. Nanoemulsion, hergestellt nach zumindest einem Verfahren gemäß den Ansprüchen 6 bis 16, **dadurch gekennzeichnet, dass** sie lagerstabil für mindestens 48 Stunden ist.

19. Verwendung einer ein oder mehrere Pestizide enthaltenden Nanoemulsion, nach einem der Ansprüche 7 bis 18 in Pflanzenschutz- und/oder Schädlingsbekämpfungsmittelformulierungen.

20. Verwendung einer ein oder mehrere Pestizide enthaltenden Nanoemulsion nach einem der Ansprüche 7 bis 18 in Formulierungen zur Mitverwendung in veterinärischen und/oder pharmazeutischen und/oder kosmetischen Zubereitungen.
